(19) Europäisches Patentamt

European Patent Office

Office européen des brevets

(11) **EP 2 011 836 B1**

(12) **EUROPEAN PATENT SPECIFICATION**

(45) Date of publication and mention
of the grant of the patent:
**06.03.2013 Bulletin 2013/10**

(21) Application number: **07739938.4**

(22) Date of filing: **27.03.2007**

(51) Int Cl.:
*C09C 3/00* (2006.01)      *A61K 8/25* (2006.01)
*C09C 1/00* (2006.01)      *C09C 1/28* (2006.01)
*C09D 5/29* (2006.01)      *C09D 7/12* (2006.01)
*C09D 11/00* (2006.01)      *C09D 201/00* (2006.01)

(86) International application number:
**PCT/JP2007/056500**

(87) International publication number:
**WO 2007/116769 (18.10.2007 Gazette 2007/42)**

(54) **BRIGHT PIGMENT WITH GOLD TONE, AND COSMETIC, PAINT, INK OR RESIN COMPOSITION CONTAINING THE SAME.**

GLAENZENDES PIGMENT MIT GOLDTON UND ES JEWEILS ENTHALTENDES KOSMETIKUM, ANSTRICHZUSAMMENSETZUNGEN, TINTEN- ODER HARZZUSAMMENSETZUNGEN.

PIGMENT DE BRILLANCE À TONALITÉ DORÉE ET PRÉPARATION COSMÉTIQUE, MATÉRIAU D'ENROBAGE, ENCRE OU COMPOSITION À BASE DE RÉSINE CONTENANT LEDIT PIGMENT

(84) Designated Contracting States:
**DE FR GB IT**

(30) Priority: **27.03.2006 JP 2006084752**

(43) Date of publication of application:
**07.01.2009 Bulletin 2009/02**

(73) Proprietor: **Nippon Sheet Glass Company, Limited Tokyo 108-6321 (JP)**

(72) Inventor: **KITAMURA, Takeaki Tokyo, 108-6321 (JP)**

(74) Representative: **Hall, Matthew Benjamin Dehns
St. Bride's House
10 Salisbury Square
London EC4Y 8JD (GB)**

(56) References cited:
**EP-A1- 1 707 599      EP-A2- 0 220 617
WO-A1-01/40383      JP-A- 10 088 027
JP-A- 62 100 770      JP-A- 2002 138 010
JP-A- 2004 204 209      US-A1- 2003 072 961**

**Description**

Technical Field

The present invention relates to a bright pigment with a gold tone, and also relates to a cosmetic, a paint, an ink, a resin composition or the like containing the pigment.

Background Art

Conventionally, scaly aluminum particles, graphite flake particles, scaly glass particles, scaly glass particles covered with silver and mica flake particles covered with a metal oxide such as titanium dioxide or iron oxide are known as bright pigments.

These bright pigments have the characteristics that their surfaces reflect light and glitter, and are used as an ingredient for paints, inks, resin compositions, cosmetics and so on. By using these bright pigments, a coated surface obtained through application of a paint, a printed surface using an ink or the surface of a molded resin product molded using a resin composition can be provided with varied, highly attractive and distinctive appearances combined with the color tones of their substrates.

The following are known as bright pigments with metallic luster.

(1) Aluminum powder or powder obtained by covering a resin foil with metal and pulverizing the foil
(2) Mica powder provided with a metal coating

Since these bright pigments have metallic luster, they can give high brightness to objects in which they are used, and can make the objects appear to be well designed.

For this reason, bright pigments are mixed with paints used for coating cars, motorcycles, OA equipment, mobile phones, home electrical appliances, etc., inks used for various printed matter, writing materials, etc., or cosmetics, and used in a wide range of applications.

In particular, bright pigments with a gold tone show a color that can provide a high-quality appearance, and thus preferably are used in such various applications.

For example, METASHINE (registered trademark) GG already has been marketed by the present applicant as a bright pigment with a gold tone. In this bright pigment, a flaky glass substrate is covered with silver and further is covered with gold. A cosmetic containing a flaky glass substrate covered with metal also has been proposed (for example, see JP 2002-138010 A).

A golden pigment in which a substrate such as mica is covered with a metal oxide also is disclosed (for example, see JP H9-012919 A). In this golden pigment, the substrate is covered with a carbon-doped titanium dioxide layer and an iron(III) oxide layer in this order.

A pearlescent cosmetic pigment in which a glass flake is covered with a titanium dioxide coating and an iron oxide coating in this order also is disclosed (for example, see JP 2004-352725 A). A bright pigment for coloring is also disclosed in which flaky glass powder is covered with a metallic coating layer, and the metallic coating layer is covered with a silica-based coating containing an inorganic or organic pigment (for example, see WO 2005/071019).

WO 01/40383 A1 and US 2003/0072961 A1 disclose precious metal color effect materials composed of a plurality of encapsulated substrate platelets.

Disclosure of Invention

Problem to be Solved by the Invention

Conventionally, in order to obtain a color tone like pure gold for a bright pigment having a gold coating, the thickness of the gold coating had to be at least 10 nm to 20 nm. This inevitably leads to an increase in the price of the bright pigment with a gold tone, and therefore, the application of such a bright pigment is limited because of cost.

Another problem is that when a color tone like pure gold is to be obtained for a bright pigment having an oxide coating, the desired color tone could not be obtained depending on the angle of view.

The present invention provides a bright pigment that can give a color tone like pure gold when the thickness of its gold coating is smaller than that of conventional bright pigments, and a cosmetic, a paint, an ink, a resin composition or the like containing the pigment.

Means for Solving Problem

A bright pigment with a gold tone according to the present invention is defined in claim 1.

A cosmetic according to the present invention contains the bright pigment with a gold tone according to the present invention.

A paint according to the present invention contains the bright pigment with a gold tone according to the present invention.

An ink according to the present invention contains the bright pigment with a gold tone according to the present invention.

A resin composition according to the present invention contains the bright pigment with a gold tone according to the present invention.

Brief Description of Drawings

[FIG. 1] FIG. 1 is a schematic cross-sectional view of an example of a bright pigment with a gold tone according to the present invention.

[FIG. 2] FIG. 2 is a schematic cross-sectional view of another example of a bright pigment with a gold tone according to the present invention.

[FIG. 3] FIG. 3 is a conceptual diagram illustrating a method for measuring a color tone.

[FIG. 4] FIG. 4 is a conceptual diagram illustrating measurement of a flop index.

Description of the Invention

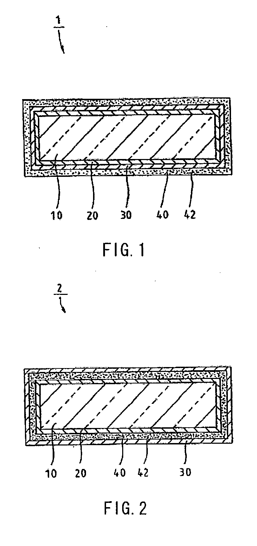

As shown in FIG. 1, an example of a bright pigment with a gold tone (hereinafter, simply may be referred to as "bright pigment") of the present invention includes a scaly glass substrate 10, a base coating 20, a gold coating 30 and a yellow coating 40. The scaly glass substrate 10 is covered with the base coating 20, the gold coating 30 and the yellow coating 40 in this order. As shown in FIG. 2, in another example of the bright pigment of the present invention, the scaly glass substrate 10 is covered with the base coating 20, the yellow coating 40 and the gold coating 30 in this order.

Applications

Since a bright pigment of the present invention is highly safe and stable, and has a gold tone, the pigment may be used for various applications, by being added, for example, to cosmetics, inks, paints, resin compositions or the like.

Examples of the above-mentioned cosmetics include facial cosmetics, makeup cosmetics and hair cosmetics. In particular, a bright pigment of this embodiment may be suitable for use in makeup cosmetics, including, for example, foundation such as makeup base and face powder, eye shadow, nail color, eyeliner, mascara, lip stick and lame powder. Although there is no particular limitation with respect to the form of the cosmetics, the cosmetics may be in the form of powder, a cake, a pencil, a stick, ointment, liquid, milky lotion and cream, for example.

Examples of the objects on which printing is performed using the above-mentioned ink include a wrapping package, wrapping paper, wallpaper, cloth, a decorative sheet, textile, various films and labels.

Examples of the fields in which the above-mentioned paint can be used include the bodies of cars, motorcycles, bicycles and the like, building materials, roofing tiles, furniture, household articles, containers, stationery and sporting goods.

Examples of the molded products molded using the above-mentioned resin composition include containers for cosmetics, food containers, wall covering materials, flooring materials, casings for home electronics, accessories, stationery, toys, bathtubs, bath-related items, footwear, sporting goods and toilet articles.

Aside from the above-described applications, a bright pigment of this embodiment also can be used as material for watercolors, oil colors, synthetic leather, textile printing, buttons, lacquer ware and pottery pigments, for example.

Scaly Glass Substrate

The shape of the scaly glass substrate 10 may vary depending on the application, and is not particularly limited. Generally, it is preferable that the average particle diameter is 1 $\mu$m to 500 $\mu$m, and the average thickness is 0.3 $\mu$m to 10 $\mu$m. When the average particle diameter is too large, there is a possibility that the scaly glass substrate 10 may be crushed during blending of the bright pigment with a paint, a resin composition, or the like. On the other hand, when the average particle diameter is too small, the principal plane of the bright pigment (particles) is oriented randomly in the resulting coating film or a molded resin product, and light reflected from the individual particles is weakened. Accordingly, the brightness will be impaired. When the average particle diameter is 1 $\mu$m to 500 $\mu$m, it is possible to prevent the bright pigment from being crushed during the blending process, and to increase the brightness.

In the case where an example of the bright pigment according to the present invention is used, for example, for a cosmetic, it may be sufficient that the average particle diameter of the scaly glass substrate 10 is suitably selected according to the kind or the like of the cosmetic. In general, the average particle diameter, however, is preferably 20 $\mu$m to 500 $\mu$m, more preferably 20 $\mu$m to 250 $\mu$m. When the average particle diameter is 20 $\mu$m to 500 $\mu$m, it is possible to achieve a cosmetic whose particles have strong brightness and hence stronger glitter.

The average thickness of the scaly glass substrate 10 is preferably 0.5 $\mu$m to 7 $\mu$m, more preferably 0.5 to 3 $\mu$m. An average thickness of 0.5 $\mu$m to 7 $\mu$m makes it possible to achieve good expansibility and good usability of a cosmetic containing the bright pigment of the present invention on the surface of keratin, which constitutes the stratum corneum of skin, nail, lip and the like, and is therefore preferable.

In the case where an example of the bright pigment of the present invention is used, for example, for an ink, the average particle diameter of the scaly glass substrate 10 is preferably 1 $\mu$m to 40 $\mu$m, more preferably 15 to 30 $\mu$m. For example, in the case where the ink is printed by a method such as gravure printing, it is possible to achieve favorable printability in terms of, for example, plate fogging when the average particle diameter of the bright pigment is 1 $\mu$m to 40 $\mu$m. It should be noted that "plate fogging" is a phenomenon in which a doctor blade cannot scrape off the ink on a printing block sufficiently at the time of printing, and the remaining ink is transferred to the printed object, thereby causing smudging on the printed matter.

The average thickness of the scaly glass substrate 10 is preferably 0.3 $\mu$m to 3 $\mu$m. An average particle diameter of 0.3 $\mu$m to 3 $\mu$m can achieve excellent finished quality on the surface of the print.

Although there is no particular limitation with respect to the method for manufacturing the scaly glass substrate 10, blow molding is preferred, for example. In blow molding, raw material cullet is melted first. The melted glass is discharged continuously from a circular slit, and a gas such as air is blown simultaneously from a blow nozzle provided on the inner side of the circular slit. Thereby, the melted glass is swollen, while being pulled, into the shape of a balloon. By pulverizing this balloon-shaped glass, a scaly glass substrate 10 is obtained.

A scaly glass substrate 10 manufactured by the above-described blow molding has a smooth surface, and thus reflects light well. Blending an example of the bright pigment using such a scaly glass substrate 10 with a paint, a resin composition or the like can achieve a coated surface, a molded resin product or the like having high brightness, and therefore is preferable. Examples of commercial products of such a scaly glass substrate 10 include MICROGLAS (registered trademark) GLASFLAKE (registered trademark) series (RCF-160, REF-160, RCF-015, REF-015) manufactured by Nippon Sheet Glass Co., Ltd.

Base Coating

The base coating 20 contains at least one metallic material selected from the group consisting of silver, nickel and a nickel-based alloy, as a principal component. In the case where the base coating 20 contains a simple substance of silver, the base coating 20 may be formed substantially from silver. In the case where the base coating 20 contains a simple substance of nickel, the base coating 20 may be formed substantially from nickel.

When the total mass of the bright pigment of the present invention is taken as 100 mass%, the above-mentioned at least one metallic material selected from the group consisting of silver, nickel and a nickel-based alloy is contained in an amount of preferably not less than 10 mass%, more preferably not less than 15 mass%, still more preferably not less than 20 to 25 mass%.

As the nickel-based alloy, it is preferable to use, for example, a nickel-phosphorus alloy, a nickel-tungsten-phosphorus alloy, a nickel-iron-tungsten-phosphorus alloy, a nickel-cobalt alloy, a nickel-cobalt-phosphorus alloy, a nickel-palladium-phosphorus alloy, a nickel-tin-phosphorus alloy or a nickel-copper-phosphorus alloy. A nickel-based alloy has higher water resistance and higher corrosion resistance than a simple substance of nickel. Therefore, when the base coating 20 contains a nickel-based alloy, an ink, a paint or the like containing the bright pigment of the present invention will have improved water resistance and corrosion resistance. However, the content of nickel in the nickel-based alloy is preferably not less than 60 atom%, preferably not less than 70 atom%, from the viewpoint of securing high brightness.

In the bright pigment of the present invention, the scaly glass substrate 10 is covered with the above-described base coating 20, so that the quantity of reflected light increases and the brightness of the bright pigment is enhanced.

There is no particular limitation with respect to the method for covering the scaly glass substrate 10 with the base coating 20, and any known film formation method can be used.

Specific examples of the commercial products of a scaly glass substrate having a silver coating formed on its surface and exhibiting brightness include MICROGLAS (registered trademark) METASHINE (registered trademark) PS series (MC2080PS, MC5480PS, MC5230PS, MC5150PS, MC5090PS, MC5030PS, ME2040PS, ME2025PS) manufactured by Nippon Sheet Glass Co., Ltd.

Examples of the commercial products of a scaly glass substrate having a nickel-phosphorus alloy coating formed on its surface and exhibiting the same brightness as the stated scaly glass substrate having a silver coating formed on its surface include MICROGLAS (registered trademark) METASHINE (registered trademark) NS series (MC5480NS, MC5230NS, MC5150NS, MC5090NS, MC5030NS) manufactured by Nippon Sheet Glass Co., Ltd.

When the base coating 20 is composed of a silver-containing base coating, which contains silver, its thickness is preferably 20 to 100 nm. When the thickness of the silver-containing base coating is 20 to 100 nm, the effect of increasing the quantity of reflected light by provision of the silver-containing base coating can be achieved sufficiently, and the cost increase due to provision of the base coating can be suppressed.

When the base coating 20 is composed of a nickel-containing base coating, which contains nickel or a nickel-based alloy, its thickness is preferably 40 to 200 nm. When the thickness of the nickel-containing base coating is 40 to 200 nm, the effect of increasing the quantity of reflected light by provision of the nickel-containing base coating can be achieved sufficiently, and the cost increase due to the provision of the base coating can be suppressed.

The base coating 20 is not limited to base coatings that are composed of a single layer as the examples shown in FIGS. 1 and 2. For example, the base coating 20 may have a two-layer structure consisting of a nickel-containing film and a silver-containing film.

Gold Coating

Although it is sufficient that the gold coating 30 contains gold, it may be composed substantially of gold. The method for forming the gold coating is not particularly limited. Examples of the formation method of the gold coating include sputtering, CVD and electroless (chemical) plating. Among them, electroless plating is preferable in that a uniform film can be formed easily.

The thickness of the gold coating 30 is preferably 0.5 to 5 nm, more preferably 1 to 3 nm, still more preferably 1 to 2 nm. The smaller the thickness of the gold coating 30 is, the higher the transmissivity of the gold coating 30 to visible light becomes. Light that has been transmitted through the gold coating 30 is reflected at the base coating 20, and the light reflected at the base coating 20 is refracted at and/or scattered about the interface, thereby increasing the brightness of the bright pigment. However, when the thickness of the gold coating 30 is too small, the gold tone of the bright pigment will be impaired. When the thickness of the gold coating is 0.5 nm to 5 nm, a bright pigment with an excellent gold tone can be provided by a combination of the effect of the base coating 20 in increasing the quantity of reflected light, the color tone of the gold coating 30 and the color tone of the yellow coating. Furthermore, the above-mentioned thickness is smaller than that of gold coatings constituting conventional bright pigments, and therefore the present invention can reduce the cost of bright pigments significantly.

Yellow Coating

The yellow coating 40 contains a parent material and a yellow colorant held on the parent material. The yellow coating 40 is required to allow visible radiation to transmit therethrough. The visible radiation transmitted through the yellow coating reaches the base coating or the gold coating, and is reflected there. This reflection allows the bright pigment to exhibit high brightness.

The raw material of the parent material that allows transmission of visible radiation include at least one selected from the group consisting of aluminum hydroxide, zirconium hydroxide, calcium hydroxide, hydrated aluminum oxide, hydrated zirconium oxide, hydrated calcium oxide, silica and hydrotalcite.

In particular, at least one material selected from the group consisting of aluminum hydroxide, hydrated aluminum oxide, zirconium hydroxide and hydrated zirconium oxide is preferable. These materials are preferred as a material that allows a water-soluble yellow colorant, which will be described later, to be laked with Al and/or Zr. In addition, these materials have very high transparency, and therefore also are preferred as a material that covers the scaly glass substrate.

The yellow colorant is at least one of the following water-soluble colorants or water-insoluble colorants.

Water-Soluble colorant

The water-soluble colorants are the following Yellow acid colorants prescribed by Color Index Generic Name (C. I. G. N): Acid Yellow 23 (certified colorant name in Japan: Yellow No. 4, hereinafter the name in the parenthesis indicates the certified colorant name in Japan), Food Yellow 3 (Yellow No. 5), Acid Yellow 73 (Yellow No. 202 (1), Yellow No. 202 (2)), Acid Yellow 3 (Yellow No. 203), Acid Yellow 40 (Yellow No. 402), Acid Yellow 1 (Yellow No. 403), Acid Yellow 36 (Yellow No. 406) and Acid Yellow 11 (Yellow No. 407).

Water-insoluble colorant

The water-insoluble colorants are the following yellow pigments prescribed by C. I. G. N. above: Acid Yellow 73 (Yellow No. 201), Solvent Yellow 33 (Yellow No. 204), Pigment Yellow 12 (Yellow No. 205), Pigment Yellow 1 (Yellow No. 401), Solvent Yellow 5 (Yellow No. 404), Solvent Yellow 6 (Yellow No. 405), Pigment Yellow 110, Pigment Yellow 139, Pigment Yellow 150 and Pigment Yellow 154.

There is no particular limitation with respect to the formation method of the yellow coating containing a yellow colorant, and any known method may be used. Some examples of the formation method of the yellow coating shall be described below.

Formation Method of Yellow Coating Containing Water-Soluble colorant

In an example of the formation method of the yellow coating containing a water-soluble colorant described above, first, a coating object is covered with, for example, a hydroxide such as aluminum hydroxide or zirconium hydroxide to form a parent material. Subsequently, after allowing the water-soluble colorant to permeate through the parent material, the water-soluble colorant is laked. The permeation of the water-soluble colorant through the parent material can be performed, for example, by making the scaly glass substrate covered with the parent material into slurry with water, and adding the water-soluble colorant to this slurry. The details of this method, by which the water-soluble colorant is laked by heating the slurry to which the water-soluble colorant is added, are disclosed in DE 2429762, US 4,084,983, JP 2002-188023 A and JP 2002-194247 A, for example.

According to the above-mentioned documents, aluminum hydroxide can be obtained, for example, through a reaction of an acidic aluminum compound with a suitable alkali. Alternatively, aluminum hydroxide also can be obtained through a reaction of an alkaline aluminum compound with a suitable acid. By carrying out either of these reactions in a suspension obtained by adding the coating object containing the scaly glass substrate to water, aluminum hydroxide will be deposited on the surface of the scaly glass substrate to form a parent material composed of aluminum hydroxide.

Examples of the acidic aluminum compound include aluminum salts of water-soluble mineral acid, such as aluminum chloride, aluminum sulfate, aluminum nitrate and aluminum phosphate. Examples of the alkaline aluminum compound include alkali metal aluminates such as sodium aluminate. Suitable examples of alkalis include a sodium hydroxide aqueous solution and an ammonium hydroxide aqueous solution. Suitable examples of acids include hydrochloric acid, sulfuric acid and nitric acid.

The amount of the alkali or acid used may be determined such that a sufficient amount of aluminum hydroxide can be deposited on the surface of the scaly glass substrate. During the above-described reactions, the pH of the above-mentioned suspension is maintained constantly within the range of 3 to 9, and the temperature of the suspension is maintained within the range of 30 to 100°C, preferably 40 to 75°C. The suspension whose pH and temperature are maintained is stirred for 5 minutes to 4 hours, preferably 30 minutes to 2 hours.

There are no particular limitations with respect to a zirconium source as long as it is a zirconium salt, and the preferred specific examples include zirconium compounds such as zirconium oxochloride, zirconium oxosulfate, zirconium oxonitrate, zirconium oxoacetate and zirconium alkoxide. Although the zirconium source is more preferably a water-soluble zirconium compound, it also may be a slurry solution containing a solid-state zirconium hydroxide that can be obtained by hydrolyzing a zirconium compound. Examples of the above-stated slurry solution include a low water-soluble slurry solution of a zirconium hydroxide that is obtained by hydrolyzing an aqueous solution of zirconium oxonitrate using ammonium hydroxide or the like.

Next, the formation method of the parent material shall be described in an exemplary case in which the source material of the parent material is hydrotalcite.

Hydrotalcite is a lamellar crystal compound in the form of a divalent-trivalent composite metal hydroxide, and has anion exchanging properties.

The composition of a hydrotalcite compound coating containing a water-soluble yellow dye (acid colorant) is represented by the following general formula (oxide representation).

$$M^1_{1-x} \cdot M^2_x(OH)_2 \cdot X_{2/n} \cdot mH_2O$$

wherein x represents a number in the range of $0.10 \leq x \leq 0.75$, X represents a monovalent or divalent acid colorant, n represents a valence of 1 or 2, and m represents a number in the range of $0 \leq m \leq 30$. $M^1$ is at least one divalent metal selected from Mg, Mn, Fe, Co, Ni, Cu and Zn, and $M^2$ is at least one trivalent metal selected from Al, Cr, Mn, Fe, Co and Ga.

A hydrotalcite compound coating containing a yellow dye (acid colorant) may be formed as follows, for example.

A suspension is prepared by adding a salt of $M^1$, a salt of $M^2$, an coating object and an acid colorant to water. Examples of the coating object include a scaly glass substrate covered with a base coating and a scaly glass substrate covered with a base coating and a gold coating. This suspension is neutralized, for example, with alkali. Then, the coating object is covered with hydrotalcite, and the acid colorant is intercalated in that hydrotalcite serving as a parent material. The details of this method are disclosed in JP 2001-234090 A and JP 2003-213156 A, for example.

Examples of the salt of $M^1$ include water-soluble salts such as chlorides, sulfates and nitrates of $M^1$. Examples of the salt of $M^2$ include water-soluble salts such as chlorides, sulfates and nitrates of $M^2$. Examples of the above-mentioned alkali include sodium hydroxide, potassium hydroxide and ammonium hydroxide.

The thickness of the yellow coating containing a water-soluble colorant described above is preferably 10 nm to 100 nm. When the thickness of the yellow coating is 10 nm to 100 nm, the yellow colorant can be contained in a suitable amount, so that suitable color tone and brightness can be achieved for the bright pigment.

The content of the yellow colorant in the yellow coating is preferably 0.1 to 10 mass%, more preferably 0.5 to 5 mass%, still more preferably 1 to 3 mass%, relative to the total mass of the bright pigment as 100 mass%.

Formation Method of Yellow Coating Containing Water-Insoluble colorant

In an example of the formation method of the yellow coating containing a water-insoluble colorant, an coating object first is dispersed in a coating solution containing an organometallic compound that can be hydrolyzed and polycondensed and a water-insoluble colorant. The coating object is a scaly glass substrate covered with a base coating and a gold coating in this order, or a scaly glass substrate covered with a base coating. Then, all are stirred continuously for 1 to 15 hours, thereby hydrolyzing and polycondensing the organometallic compound. By doing so, a coating containing a water-insoluble colorant can be formed on the surface of the scaly glass substrate. The amount of the scaly glass substrate covered with the base coating and the gold coating in this order, that is added in the coating solution preferably may be decided so as to be 1 to 30 mass%, relative to the total mass of the bright pigment as 100 mass%. Since the mass of the gold coating is negligible, the amount of the scaly glass substrate covered with the base coating that is added in the coating solution also preferably may be decided so as to be 1 to 30 mass%, relative to the total mass of the bright pigment as 100 mass%.

Subsequently, the scaly glass substrate covered with the yellow coating is filtered, washed with water, and dried. The heat treatment for drying preferably is performed at room temperature to 200°C for 1 to 5 hours.

Preferred specific examples of the organometallic compound include an organosilicon compound, an organotitanium compound, an organozirconium compound and an organoaluminium compound. In particular, an organosilicon compound is preferable because of its high transmissivity to visible radiation. If an organometallic compound contains an organosilicon compound, then a silica-based coating will be formed as the parent material. The details of this method are disclosed in JP H5-171055 A, JP H7-133211 A and JP 2006-176742 A, for example.

The thickness of the yellow coating is not particularly limited, and is preferably 10 nm to 500 nm from the viewpoint of ensuring an excellent color tone, reliably holding the water-insoluble colorant, ensuring a high surface smoothness of the coating, and reducing the cost.

Although it is sufficient that the silica-based coating contains silica ($SiO_2$) as a principal component, it may be composed substantially only of silica. The silica-based coating may contain titania, zirconia or the like for adjustment of the refractive index of the coating, and improvement in alkali resistance of the coating.

Preferred specific examples of the organosilicon compound that can be hydrolyzed and polycondensed, contained in the coating solution include: tetraalkoxysilanes such as tetramethoxysilane, tetraethoxysilane, tetrapropoxysilane and tetrabutoxysilane; and methyltrialkoxysilanes such as methyltrimethoxysilane, methyltriethoxysilane, methyltripropoxysilane and methyltributoxysilane.

Among them, those having a comparatively low molecular weight, including, for example, tetraalkoxysilane having an alkoxyl group with not more than three carbon atoms is preferable because of ease of formation of a dense coating. Further, a polymer of tetraalkoxysilane with an average degree of polymerization of not more than 5 is more preferable.

The coating solution preferably contains 0.5 to 20 mass% of the organosilicon compound, and 0.01 to 5 mass% of the water-insoluble colorant, for example. In addition to these, the coating solution contains water for hydrolysis and an alcohol-based solvent for hydrolysis.

The content of water in the coating solution is preferably 0.1 to 20 mass%.

As the alcohol-based solvent, it is preferable to use, for example, methyl alcohol, ethyl alcohol, 1-propyl alcohol, 2-propyl alcohol, butyl alcohol or amyl alcohol. A chain saturated monovalent alcohol with not more than three carbon atoms is more preferable because of its high evaporation rate at normal temperature. Examples of the chain saturated monovalent alcohol with not more than three carbon atoms include methyl alcohol, ethyl alcohol, 1-propyl alcohol and 2-propyl alcohol. The content of the above-mentioned alcohol-based solvent in the coating solution is preferably 50 to 98 mass%, for example.

The coating solution may contain a catalyst. As the catalyst, alkali catalysts such as ammonium hydroxide and sodium hydroxide are preferable from the viewpoint of promoting the hydrolysis and polycondensation of the organosilicon compound and allowing an excellent yellow coating to be formed for each particle (a scaly glass substrate covered with a base coating and a gold coating in this order, or a scaly glass substrate covered with a base coating). The content of the catalyst in the coating solution is preferably 0.1 to 10 mass%.

The pH of the coating solution is suitably 9 to 13.

Although the average particle diameter of the water-insoluble colorant is not particularly limited, it is preferably 10 nm to 500 nm. If the average particle diameter is too small, the durability of the yellow pigment will be extremely low. On the other hand, if the average particle diameter is too large, the degree of shielding of reflected light by the yellow pigment will be large, which reduces the brightness and also degrades the color tone.

The content of the water-insoluble colorant is preferably not more than 2.0 mass%, more preferably less than 1.0 mass%, still more preferably 0.01 to 0.8 mass%, relative to the total mass of the bright pigment of the present invention as 100 mass%.

Next, an embodiment of the present invention will be described in detail by way of examples and comparative examples.

Various average particle diameters were measured using a laser diffraction-type grain size meter.

The average thickness of the scaly glass substrate was determined by measuring the thicknesses of 100 grains of the scaly glass substrates, and calculating their average. The thickness of each scaly glass substrate was determined by measuring the optical path difference between direct light (light that has not been affected by a phase object), and light that has been transmitted through the scaly glass substrate, using an interference microscope.

The thickness of each coating was measured using a secondary ion-microprobe mass spectrometer (SIMS). Specifically, the thickness was determined based on the component distribution from the surface of the bright pigment to the surface of the scaly glass substrate.

Examples

Example 1

A bright pigment of Example 1 has a structure in which a scaly glass substrate is covered with a base coating (silver coating), a gold coating and a yellow coating in this order, as in the example shown in FIG. 1. The parent material constituting the yellow coating is composed of silica. The bright pigment of Example 1 was produced as follows.

Scaly Glass Substrate Covered with Silver Coating

First, MC2080PS manufactured by Nippon Sheet Glass Co., Ltd. was provided as a scaly glass substrate covered with a silver coating. This has a structure in which the scaly glass substrate is covered with a silver coating (thickness: 20 to 100 nm) by electroless plating.

Formation of Gold Coating

The gold coating was formed by electroless plating in the following manner. First, 6 g of sodium L-ascorbate serving as a reducing agent and 3 g of disodium hydrogenphosphate serving as a pH adjuster were added to 300 mL of pure water, and all were heated to 60°C while being stirred. To the resulting solution, 13 g of a gold sodium sulfite aqueous solution (concentration: 50 g/L) was added as a raw material of gold, thereby obtaining a gold plating liquid.

Next, 30 g of a scaly glass substrate covered with a silver coating (MC2080PS manufacture by Nippon Sheet Glass Co., Ltd.) was added to the gold plating liquid. Then, all were stirred for 20 to 30 minutes, and a gold coating with an average thickness of 1.4 nm was formed on the silver coating through an electroless plating reaction.

Subsequently, after filtration and washing with water were repeated several times, the scaly glass substrate covered with the silver coating and the gold coating was dried by being heat-treated for 2 hours at 180°C. The scaly glass substrate covered with the silver coating and the gold coating had an average particle diameter of 80 $\mu$m, and an average thickness of 1.3 $\mu$m, and showed metallic luster with a tinge of pale yellow.

Formation of Yellow-Pigment-Dispersed Silica Coating

Next, a silica coating in which a water-insoluble yellow pigment was dispersed as a yellow coating on a gold coating by a sol gel process was formed as follows.

15 mL of tetraethoxysilane, 300 mL of isopropyl alcohol (IPA) and 60 mL of pure water were mixed. To the resulting mixed solution, 2.0 g of a solution containing 10 mass% of fine particles of Pigment Yellow 110 expressed by C. I. G. N. (yellow pigment, average particle diameter: about 100 nm) were added, and mixed, thereby obtaining a silica coating formation solution.

To this silica coating formation solution, 30 g of the scaly glass substrate covered with the silver coating and the gold coating was added, and all were stirred and mixed together using a stirrer. Subsequently, after 14 mL of an ammonium hydroxide solution (concentration: 25 mass%) was added to the silica coating formation solution, the silica coating formation solution was stirred for two to three hours, thereby causing a dehydration condensation reaction. This made it possible for a silica coating in which the yellow pigment was dispersed to be deposited uniformly on the surface of the scaly glass substrate in the silica coating formation solution.

Subsequently, after filtration was performed, the scaly glass substrate covered with the silver coating, the gold coating and the silica coating was dried by being heat-treated for 2 hours at 180°C. Thus, a bright pigment including the silica coating (thickness: 100 nm) in which the yellow pigment was dispersed was obtained. This bright pigment exhibited a color tone of pure gold.

Example 2

A bright pigment of Example 2 has a structure in which a scaly glass substrate is covered with a base coating (silver coating), a yellow coating and a gold coating in this order, as the example shown in FIG. 2. MC2080PS was used as a scaly glass substrate covered with a silver coating, as in Example 1. The formation methods of the yellow-pigment-dispersed silica coating and the gold coating, etc. were the same as those in Example 1.

Example 3

A bright pigment of Example 3 has a structure in which a scaly glass substrate is covered with a base coating (silver coating), a gold coating and a yellow coating in this order, as the example shown in FIG. 1. The bright pigment of Example 3 was the same as the bright pigment of Example 1, except for the use of a water-soluble yellow dye as the yellow colorant contained in the yellow coating and aluminum hydroxide as the source material of the parent material constituting the yellow coating.

First, MC2080PS manufactured by Nippon Sheet Glass Co., Ltd. was used as a scaly glass substrate covered with a silver coating as in Example 1, and a gold coating with an average thickness of 1.4 nm was formed on the silver coating through an electroless plating reaction. Subsequently, after filtration and washing with water were repeated several times, the scaly glass substrate covered with the silver coating and the gold coating was dried by being heat-treated for 2 hours at 180°C. The scaly glass substrate covered with the silver coating and the gold coating had an average particle diameter of 80 $\mu$m and an average thickness of 1.3 $\mu$m, and showed metallic luster with a tinge of pale yellow.

Next, a 10 mass% aluminum (III) nitrate nonahydrate [$Al(NO_3)_3 \cdot 9H_2O$] aqueous solution was produced. Meanwhile, about 30 g of the scaly glass substrate covered with the silver coating and the gold coating was added to 0.3 L of purified water to obtain a suspension. The temperature of the obtained suspension was kept at 75°C with a water bath, and the pH of the suspension was adjusted to 6 using dilute nitric acid. Subsequently, to this suspension, 20 g of the above-mentioned aluminum (III) nitrate nonahydrate aqueous solution and 8 g of a sodium hydroxide aqueous solution (10 mass%) were slowly added over 10 minutes. After addition of these aqueous solutions, the suspension was stirred continuously for 30 minutes, while still keeping the temperature of the suspension at 75°C and also maintaining the pH at 6. Thereafter, the suspension was filtered with filter paper to give a filtrated solid matter, which then was washed with water, thereby obtaining a scaly glass substrate in which the silver coating, the gold coating and the aluminum hydroxide coating were formed in this order.

Subsequently, about 30 g of the scaly glass substrate in which the silver coating, the gold coating and the aluminum hydroxide coating were formed was added to 0.3 L of purified water. With the temperature of the obtained suspension kept at 70°C with a water bath, 14 g of a solution containing 10 mass% of Acid Yellow 23 (Yellow No. 4) was added to the suspension. After addition of this solution, the suspension was stirred continuously for the 15 minutes, while still maintaining the temperature of the suspension at 70°C and the pH at 6. Thereafter, the suspension was filtrated with filter paper to give a filtrated solid matter, which was then dried, and heat-treatment was performed for the filtration solid matter for two hours at 120°C. Thus, a bright pigment including aluminum hydroxide (parent material, thickness: 30 nm) in which the yellow dye was dispersed was obtained. This bright pigment exhibited a color tone of pure gold.

Example 4

A bright pigment of Example 4 has a structure in which a scaly glass substrate is covered with a base coating (nickel-phosphorus alloy coating, nickel atom%: 85), a gold coating and a yellow coating in this order, as in the example shown in FIG. 1. In the bright pigment of Example 4, the base coating is the nickel-phosphorus alloy coating, the yellow colorant contained in the yellow coating is a water-insoluble yellow pigment, and the raw material of the parent material constituting the yellow coating is silica. Except for these, the bright pigment of Example 4 is the same as the bright pigment of Example 1.

First, MC5150NS (average particle diameter: 150 $\mu$m, average thickness: 5.0 $\mu$m) manufactured by Nippon

Sheet Glass Co., Ltd. was provided as a scaly glass substrate covered with a nickel-phosphorus alloy coating. MC5150NS manufactured by Nippon Sheet Glass Co., Ltd. is a scaly glass substrate covered a nickel-phosphorus alloy coating (thickness: about 150 nm) by electroless plating.

Formation Of Gold Coating

The gold coating was formed by electroless plating in the following manner. First, 3 g of sodium L-ascorbate serving as a reducing agent and 1.5 g of disodium hydrogenphosphate serving as a pH adjuster were added to 300 mL of pure water, and all were heated to 60°C, while being stirred, to be dissolved. To the resulting solution, 6.5 g of an aqueous solution of gold sodium sulfite (concentration: 50 g/L) was added as a source material of gold, thereby obtaining a gold plating liquid.

Next, 30 g of a scaly glass substrate covered with a nickel-phosphrous alloy coating (MC5150NS manufacture by Nippon Sheet Glass Co., Ltd.) was added to the gold plating liquid. While stirring the whole for 20 to 30 minutes, a gold coating with an average thickness of 2.8 nm was formed on the nickel-phosphorus alloy coating through an electroless plating reaction.

Subsequently, after filtration and washing with water were repeated several times, the scaly glass substrate covered with the nickel-phosphorus alloy coating and the gold coating was dried by being heat-treated for 2 hours at 180°C. The scaly glass substrate covered with the nickel-phosphorus alloy coating and the gold coating had an average particle diameter of 150 $\mu$m and an average thickness of 5 $\mu$m, and showed metallic luster with a tinge of pale yellow.

Formation of Yellow-Pigment-Dispersed Silica Coating

Next, a silica coating in which a water-insoluble yellow pigment was dispersed as a yellow coating on the gold coating by a sol-gel process was formed in the following manner.

3.8 mL of tetraethoxysilane, 300 mL of isopropyl alcohol (IPA) and 12 mL of pure water were mixed. To the resulting mixed solution, 0.5 g of a solution containing 10 mass% of fine particles of Pigment Yellow 110 expressed by C. I. G. N. (yellow pigment, average particle diameter: about 100 nm) were added, followed by mixing, thereby obtaining a silica coating formation solution.

To this silica coating formation solution, 30 g of the scaly glass substrate covered with the nickel-phosphorus alloy coating and the gold coating was added, and all were stirred and mixed using a stirrer. Subsequently, after 3.5 mL of an ammonium hydroxide solution (concentration: 25 mass%) was added to the silica coating formation solution, the silica coating formation solution was stirred for two to three hours, thereby causing a dehydration condensation reaction. This made it possible for a silica coating in which the yellow pigment was dispersed to be deposited uniformly on the gold coating in the silica coating formation solution.

Subsequently, after filtration and washing with water were repeated several times, the scaly glass substrate covered with the nickel-phosphorus alloy coating, the gold coating and the silica coating was dried by being heat-treated for 2 hours at 180°C. Thus, a bright pigment including the silica coating (thickness: 100 nm) in which the yellow pigment was dispersed was obtained. This bright pigment exhibited a color tone of pure gold.

Example 5

A bright pigment of Example 5 has a structure in which a scaly glass substrate is covered with a base coating (silver coating), a gold coating and a yellow coating in this order, as in the example shown in FIG. 1. The bright pigment of Example 5 was the same as the bright pigment of Example 1, except for the use of a water-soluble yellow dye as the yellow colorant contained in the yellow coating, and hydrotalcite as the source material of the parent material constituting the yellow coating.

First, MC2080PS manufactured by Nippon Sheet Glass Co., Ltd. was used as a scaly glass substrate covered with a silver coating as in Example 1, and a gold coating with an average thickness of 1.4 nm was formed on the silver coating through an electroless plating reaction. Subsequently, after filtration and washing with water were repeated several times, the scaly glass substrate covered with the silver coating and the gold coating was dried by being heat-treated for 2 hours at 180°C. The scaly glass substrate covered with the silver coating and the gold coating had an average particle diameter of 80 $\mu$m and an average thickness of 1.3 $\mu$m, and exhibited metallic luster with a tinge of pale yellow.

Next, an adjustment liquid in which 3 g of aluminum chloride hexahydrate and 5 g of magnesium chloride hexahydrate were dissolved in 50 ml of purified water was produced. Meanwhile, about 30 g of the scaly glass substrate covered with the silver coating and the gold coating, 13.5 g of an aqueous solution containing Acid Yellow 23 (10 mass%) and 0.5 g of an aqueous solution containing Food Yellow 3 (10 mass%) were added to 0.3 L of purified water, thereby obtaining a suspension. With the temperature of the obtained suspension kept at 75°C with a water bath, the whole amount of the above-described adjustment liquid was added dropwise into the suspension over 10 minutes. During dropwise addition of the adjustment liquid, the pH of the suspension was maintained at 6 using a 10 mass% sodium hydroxide aqueous solution. The suspension was stirred for 30 minutes at 90°C, with the pH still maintained at 6.

Thereafter, the suspension was filtrated with filter paper to give a filtered solid matter, which then was dried, and subjected to heat-treatment for two hours at 120°C. Thus, a bright pigment including hydrotalcite (parent material, thickness: 30 nm) containing the yellow dye was obtained. This bright pigment exhibited a color tone of pure gold with a tinge of red.

Comparative Example 1

A bright pigment of Comparative Example 1 has a structure in which a scaly glass substrate is covered with a silver coating and a yellow-pigment-dispersed silica coating in this order. The bright pigment of Comparative Example 1 is the same as the bright pigment of Example 1, except that there is no gold coating, and that the content of the yellow pigment in the silica coating formation solution is different. MC2080PS was used as a scaly glass substrate covered with a silver coating, as in Example 1. The silica coating formation solution was prepared as follows. The yellow-pigment-dispersed silica coating was formed in the same manner as that of Example 1.

15 mL of tetraethoxysilane, 300 mL of isopropyl alcohol (IPA) and 60 mL of pure water were mixed. To the resulting mixed solution, 6.0 g of a solution containing 10 mass% of fine particles of Pigment Yellow 110 (yellow pigment, average particle diameter: about 100 nm) expressed by C. I. G. N were added, followed by mixing, thereby obtaining a silica coating formation solution.

Comparative Example 2

A bright pigment of Comparative Example 2 is the bright pigment described in JP 2004-352725 A, and has a structure in which a scaly glass substrate is covered with a titanium dioxide coating and an iron oxide coating in this order.

Formation Method of Titanium Dioxide Coating

30 g of a scaly glass substrates composed of C glass (MC2080FF manufactured by Nippon Sheet Glass Co., Ltd., average particle diameter: 80 $\mu$m) was dispersed in 333 mL of distilled water. The resulting dispersion was heated to 75°C, and the pH of the dispersion was adjusted to 1.6 using diluted hydrochloric acid. Subsequently, 7 mL of an 18 mass% tin(II) chloride aqueous solution was added slowly to the dispersion, to which a 40 mass% titanium tetrachloride aqueous solution was subsequently added at a rate of 100 mL/h. During the addition of the titanium tetrachloride aqueous solution, a diluted aqueous solution of sodium hydroxide was added to the dispersion to maintain the pH of the dispersion at 1.6. A gold color resulting from interference came to be observed on the surface of the scaly glass substrate by addition of the titanium tetrachloride aqueous solution, and addition of the titanium tetrachloride aqueous solution was continued until the desired gold color had been observed. Subsequently, a scaly glass substrate in the form of slurry was filtrated from the dispersion, and the filtrated scaly glass substrate was washed with distilled water, thereby obtaining a scaly glass substrate covered with a titanium dioxide coating.

Formation Method of Iron Oxide Coating

Subsequently, the scaly glass substrate covered with a titanium dioxide coating was dispersed in 300 mL of distilled water. This dispersion was heated to 76°C, and the pH of the dispersion was adjusted to 3.2 using diluted hydrochloric acid. Subsequently, a 10 mass% iron(III) chloride aqueous solution was added to the dispersion at a rate of 0.2 mL/h. During the addition of the iron(III) chloride aqueous solution, a diluted aqueous solution of sodium hydroxide was added to the dispersion to maintain the pH of the dispersion at 3.2. Addition of the above-mentioned iron(III) chloride aqueous solution was continued until the desired gold color had been observed. Subsequently, a scaly glass substrate in the form of slurry was filtrated from the dispersion, and the filtrated solid matter was washed with distilled water. Then, the filtrated solid matter was fired at 600°C to form a $Fe_2O_3$ coating on the titanium dioxide coating. Thus, a bright pigment in which the scaly glass substrate was covered with the titanium dioxide coating and the iron oxide coating in this order was obtained.

Standard Example

A standard example is a scaly glass substrate covered with a silver coating and a gold coating (average thickness: 10 nm) in this order. Formation of the gold coating was performed using the following electroless gold plating liquid.

First, 14 g of sodium L-ascorbate serving as a reducing agent and 7 g of disodium hydrogenphosphate serving as a pH adjuster were added to 300 mL of pure water, and all were heated to 60°C to be dissolved by stirring. To the resulting solution, 91 g of a gold sodium sulfite aqueous solution (concentration: 50 g/L) was added as a source material of gold, thereby obtaining a gold plating liquid. Except for these, a gold coating with an average thickness of 10 nm was formed on the silver coating in the same manner as in Example 1.

Production of Evaluation Samples

Each of the bright pigments of the examples, the comparative examples and the standard example was blended with a clear paint composed of 30 mass% of acrylic resin and 70 mass% of an organic solvent (manufactured by Nippon Paint Co., Ltd.) at a rate of 10 mass%, and all were mixed together with a kneader. Thus, various paints were produced, and each of the paints was applied to commercially available black-and-white shielding paper so that the thickness of the coating was 9 mil (9/1000 x 25.4 mm), and the coating was air-dried. Each of the dried coatings was used as an evaluation sample.

Each of the evaluation samples was evaluated for the following points.

(1) Shielding properties
(2) Degree of metallic luster
(3) Angular dependence of reflecting color tone

(1) Shielding properties

The degree of luminance of the coatings applied to the black-and-white shielding paper was measured using a colorimeter (CR-400, manufactured by Konica Minolta Sensing, Inc.). Here, the degree of luminance (L*) was measured using the L*a*b* color system. At that time, a C light source was used as an observation light source. From the degree of luminance L* (white) of the white part and the degree of luminance L* (black) of the black part that were measured, the luminance difference ($\Delta L^* = L^*$(white) - $L^*$ (black)) was calculated, and the results are shown in Table 1. The smaller $\Delta L^*$ indicates better shielding properties.

[Table 1]

|  | Ex. 1 | Ex. 2 | Ex. 3 | Ex. 4 | Ex. 5 | Com. Ex. 1 | Com. Ex. 2 | Std. Ex. |
|---|---|---|---|---|---|---|---|---|
| L*(white) | 81.5 | 80.2 | 82.1 | 62.3 | 81.8 | 84.1 | 84.8 | 79.8 |
| L*(black) | 80.7 | 79.7 | 81.3 | 61.6 | 81.0 | 83.1 | 67.0 | 79.2 |
| $\Delta L^*$ | 0.8 | 0.5 | 0.8 | 0.7 | 0.8 | 0.8 | 17.8 | 0.6 |

As shown in Table 1, each of the examples had a small $\Delta L^*$, and therefore had excellent shielding properties, which are equivalent to those of the standard example. On the other hand, Comparative Example 2 had a large $\Delta L^*$, and therefore had shielding properties inferior to those of the standard example.

(2) Degree of metallic luster

The degree of metallic luster of the coatings applied to the black-and-white shielding paper was measured using a gloss meter (GM-O26PR, manufactured by MURAKAMI COLOR RESEARCH LABORATORY). Here, a D65 light source was used as an observation light source. Light was made incident on the coating surface at an angle of 60° or 20° from the direction perpendicular to the coating surface, and the quantity of the mirror-reflected light was measured for each case. Using the measured value of light quantity, the glossiness was calculated in accordance with ISO2813, and the results are shown in Table 2. The higher values indicate higher glossiness.

[Table 2]

|  | Ex. 1 | Ex. 2 | Ex. 3 | Ex. 4 | Ex. 5 | Com. Ex. 1 | Com. Ex. 2 | Std. Ex. |
|---|---|---|---|---|---|---|---|---|
| Luster degree (60°) | 74.3 | 78.3 | 73.3 | 68.5 | 77.7 | 59.6 | 51.6 | 80.3 |
| Luster degree (30°) | 24.6 | 26.4 | 24.2 | 20.3 | 26.5 | 18.9 | 14.6 | 27.3 |

As shown in Table 2, each of the examples had a degree of metallic luster closer to that of the standard example. On the other hand, each of Comparative Examples 1 and 2 had a degree of metallic luster smaller than that of the standard example.

(3) Angular dependence of reflecting color tone

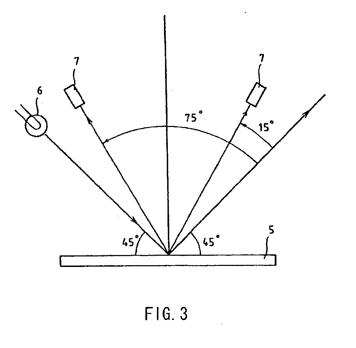

As shown in FIG. 3, an observation light source 6 was disposed in a position that allowed light to be incident on the surface of a coating 5 at an angle of 45°. A D65 light source was used as the observation light source 6. Light was emitted from the observation light source 6, and the color tone of the reflected light was measured with a detector 7.

In addition, the detector 7 was disposed in a position that allowed reception of light that had been reflected in a direction shifted by 15° from the direction of mirror reflection of the incident light (i.e., the direction of 45° with respect to the surface of the coating 5) toward the observation light side (the color tone of this reflected light is referred to as "highlight color tone"). The reason is as follows.

The quantity of reflected light that has been mirror-reflected at the bright pigment and the coating surface is too large. Therefore, coloring of the bright pigment is difficult to recognize. If the observation is performed from an angle shifted by 15° from the direction of mirror reflection, then the influence of the mirror reflection on the coating surface will be suppressed, making it easy to measure the color tone of the bright pigment resulting from reflection.

Additionally, as shown in FIG. 3, the color tone (shade color tone) of the light reflected in a direction shifted by 75° from the direction of mirror reflection toward the observation light source side was measured with the detector 7. If the observation is performed at an angle shifted by 75° from the direction of mirror reflection, then the influence of the mirror reflection will be suppressed, making it possible to measure the color tone of the bright pigment itself.

The highlight color tone and the shade color tone were measured using a multi-angle spectrophotometric colorimeter (manufactured by COLOR TECHNO SYSTEM CORPORATION). Here, L*, a* and b* were measured using the L*a*b* color system. The measurement results are shown in Table 3 (highlight color tone) and Table 4 (shade color tone).

[Table 3]

|  | Ex. 1 | Ex. 2 | Ex. 3 | Ex. 4 | Ex. 5 | Com. Ex. 1 | Com. Ex. 2 | Std. Ex. |
|---|---|---|---|---|---|---|---|---|
| L* | 135.1 | 134.4 | 135.3 | 123.9 | 136.0 | 137.7 | 114.9 | 133.6 |
| a* | 2.5 | 2.3 | 2.7 | 1.8 | 5.7 | 2.8 | 0.5 | 2.6 |
| b* | 38.9 | 37.4 | 38.4 | 34.7 | 36.7 | 38.6 | 51.1 | 39.5 |

As shown in Table 3, it was found that each of the examples achieved a highlight color tone similar to that of the standard example. On the other hand, it was found that Comparative Example 2 provided a color tone different from that of the standard example.

[Table 4]

|  | Ex. 1 | Ex.2 | Ex. 3 | Ex.4 | Ex. 5 | Com. Ex. 1 | Com. Ex. 2 | Std. Ex. |
|---|---|---|---|---|---|---|---|---|
| L* | 33.5 | 30.8 | 32.5 | 30.2 | 33.2 | 39.7 | 63.2 | 30.2 |
| a* | 1.3 | 1.5 | 1.6 | 1.8 | 4.2 | 0.14 | 4.7 | 1.8 |
| b* | 12.9 | 12.3 | 12.7 | 10.5 | 10.6 | 20.4 | 20.2 | 11.6 |

As shown in Table 4, it was found that each of the examples achieved a shade color tone similar to that of the standard example. On the other hand, each of the comparative examples provided a color tone different from that of the standard example, and the degree of the difference was particularly high in the case of Comparative Example 2.

The luminance change resulting from the observation angle can be expressed by the flop index (FI) defined by the following formula.

$$\text{FI} = 2.69 \times (L^*_{15°} - L^*_{110°})^{1.11} / (L^*_{45°})^{0.86} \qquad (1)$$

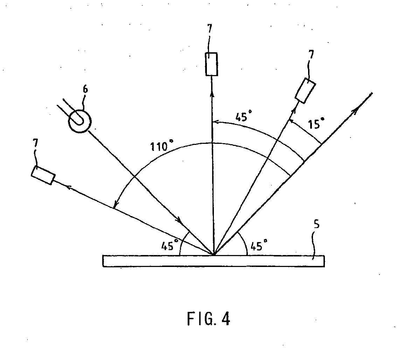

Here, $L^*_{15°}$, $L^*_{110°}$ and $L^*_{45°}$ are the degrees of luminance measured in positions tilted by 15°, 110° and 45°, respectively, toward the observation light source side, relative to the direction of mirror reflection. Each of the degrees of luminance was measured using the L*a*b* color system (see FIG. 4).

A flop index is the numerical representation of the luminance change of the highlight color tone and the shade color tone, and the degree of luminance change increases with an increase in the numerical value. If FI of a sample is the same as or similar to that of the standard example, that example can be evaluated as having a color tone like pure gold.

The above-described flop index was also measured using a multi-angle spectrophotometric colorimeter, and the results are shown in Table 5. A D65 light source was used as the observation light source.

[Table 5]

|  | Ex. 1 | Ex. 2 | Ex. 3 | Ex. 4 | Ex.5 | Com. Ex. 1 | Com. Ex. 2 | Std. Ex. |
|---|---|---|---|---|---|---|---|---|
| FI (white) | 21.0 | 21.6 | 22.1 | 20.7 | 22.1 | 15.7 | 6.7 | 22.1 |
| FI (black) | 22.1 | 22.3 | 22.5 | 21.0 | 22.8 | 17.4 | 26.7 | 23.8 |

As shown in Table 5, even in the case where the color of the base coating was either white or black, the value of the flop index of each of the examples was close to that of the standard example. On the other hand, the flop index of each of the comparative examples had a value that deviated from the that of the standard example.

As described above, the bright pigments of the examples had the same golden color tone as that of the standard example, even though the film thickness of the gold coating was less than half the thickness of the gold coating of the standard example. Therefore, the bright pigments of the examples are more advantageous in terms of cost, compared with the bright pigment of the standard example.

As described above, with the present invention, a base coating containing at least one metallic material selected from the group consisting of silver, nickel and a nickel-based alloy is provided as the base of other coatings and a yellow coating is further provided. Accordingly, it is possible to provide a bright pigment that can achieve a color tone like pure gold even if the thickness of its gold coating is reduced to be smaller than that of conventional bright pigments, and a cosmetic, a paint, an ink, a resin composition and the like that contain the bright pigment.

Industrial Applicability

The bright pigment of the present invention shows a color tone like pure gold even if the thickness of its gold coating is reduced to be smaller than that of conventional bright pigments. Accordingly, the bright pigment of the present invention can be used in a wide range of fields, and is therefore useful.

**Claims**

1. A bright pigment with a gold tone, comprising:

    a scaly glass substrate (10);
    a base coating (20) containing at least one metallic material selected from the group consisting of silver, nickel and a nickel-based alloy,
    a gold coating (30) containing gold; and
    a yellow coating (40) containing a yellow colorant and a parent material;
    wherein the yellow colorant is a water-soluble colorant or water insoluble colorant, the water-soluble colorant is at least one selected from the group consisting of Acid Yellow 23, Food Yellow 3, Acid Yellow 73, Acid Yellow 3, Acid Yellow 40, Acid Yellow 1, Acid Yellow 36 and Acid Yellow 11, which are prescribed by Color Index Generic Name, the water-insoluble colorant is at least one selected from the group consisting of Acid Yellow 73, Solvent Yellow 33, Pigment Yellow 12, Pigment Yellow 1, Solvent Yellow 5, Solvent Yellow 6, Pigment Yellow 110, Pigment Yellow 139, Pigment Yellow 150 and Pigment Yellow 154, which are prescribed by Color Index Generic Name, when the yellow colorant is a water-soluble colorant, the parent material comprises at least one material selected from the group consisting of aluminum hydroxide, zirconium hydroxide, calcium hydroxide, hydrated aluminum oxide, hydrated zirconium oxide, hydrated calcium oxide and hydrotalcite, and the water-soluble colorant permeates through the parent material in the yellow coating, when the yellow colorant is a water-insoluble colorant, the parent material comprises silica, and the water-insoluble colorant is dispersed in the parent material in the yellow coating, and

the base coating (20), the gold coating (30) and the yellow coating (40) each form a layer covering the scaly glass substrate (10), and the base coating (20) is arranged innermost among the base coating (20), the gold coating (30) and the yellow coating (40).

**2.** The bright pigment with a gold tone according to claim 1,
wherein the gold coating (30) has a thickness of 0.5 to 5 nm.

**3.** The bright pigment with a gold tone according to claim 1,
wherein the base coating (20) is a silver-containing base coating that contains silver, and
the silver-containing base coating (20) has a thickness of 20 to 100 nm.

**4.** The bright pigment with a gold tone according to claim 1,
wherein the base coating (20) is a nickel-containing base coating that contains nickel or a nickel-based alloy, and
the nickel-containing base coating has a thickness of 40 to 200 nm.

**5.** The bright pigment according to claim 1,
wherein the nickel-based alloy is a nickel-phosphorus alloy, a nickel-tungsten-phosphorus alloy, a nickel-iron-tungsten-phosphorus alloy, a nickel-cobalt alloy, a nickel-cobalt-phosphorus alloy, a nickel-palladium-phosphorus alloy, a nickel-tin-phosphorus alloy or a nickel-copper-phosphorus alloy.

**6.** The bright pigment with a gold tone according to claim 1,
wherein the scaly glass substrate (10) has an average particle diameter of 1 $\mu$m to 500 $\mu$m and an average thickness of 0.3 $\mu$m to 10 $\mu$m.

**7.** A cosmetic containing the bright pigment with a gold tone according to any of claims 1 to 6.

**8.** A paint containing the bright pigment with a gold tone according to any of claims 1 to 6.

**9.** An ink containing the bright pigment with a gold tone according to any of claims 1 to 6.

**10.** A resin composition containing the bright pigment with a gold tone according to any of claims 1 to 6.

**Patentansprüche**

**1.** Glänzendes Pigment mit einem Goldton, umfassend:

ein Schuppenglas-Substrat (10);
eine Basisbeschichtung (20), die mindestens ein metallisches Material enthält, ausgewählt aus der Gruppe bestehend aus Silber, Nickel und einer Legierung auf Nickelbasis;
eine Goldbeschichtung (30), die Gold enthält; und
eine gelbe Beschichtung (40), die ein gelbes Farbmittel und ein Grundmaterial enthält;
wobei das gelbe Farbmittel ein wasserlösliches Farbmittel oder wasserunlösliches Farbmittel ist, das wasserlösliche Farbmittel mindestens eines, ausgewählt aus der Gruppe bestehend aus Acid Yellow 23, Food Yellow 3, Acid Yellow 73, Acid Yellow 3, Acid Yellow 40, Acid Yellow 1, Acid Yellow 36 und Acid Yellow 11, die durch den Color Index Generic Name vorgeschrieben sind, ist, das wasserunlösliche Farbmittel mindestens eines, ausgewählt aus der Gruppe bestehend aus Acid Yellow 73, Solvent Yellow 33, Pigment Yellow 12, Pigment Yellow 1, Solvent Yellow 5, Solvent Yellow 6, Pigment Yellow 110, Pigment Yellow 139, Pigment Yellow 150 und Pigment Yellow 154, die durch den Color Index Generic Name vorgeschrieben sind, ist, wenn das gelbe Farbmittel ein wasserlösliches Farbmittel ist, das Grundmaterial mindestens ein Material umfasst, das ausgewählt ist aus der Gruppe bestehend aus Aluminiumhydroxid, Zirconiumhydroxid, Calciumhydroxid, hydratisiertem Aluminiumoxid, hydratisiertem Zirconiumoxid, hydratisiertem Calciumoxid und Hydrotalkit, und das wasserlösliche Farbmittel das Grundmaterial in der gelben Beschichtung durchdringt, wenn das gelbe Farbmittel ein wasserunlösliches Farbmittel ist, das Grundmaterial Siliciumdioxid umfasst und das wasserunlösliche Farbmittel in dem Grundmaterial in der gelben Beschichtung dispergiert ist, und
die Basisbeschichtung (20), die Goldbeschichtung (30) und die gelbe Beschichtung (40) jeweils eine das Schuppenglas-Substrat (10) bedeckende Schicht ausbilden und die Basisbeschichtung (20) die am weitesten innen angeordnete der Basisbeschichtung (20), der Goldbeschichtung (30) und der gelben Beschichtung (40) ist.

**2.** Glänzendes Pigment mit einem Goldton nach Anspruch 1,
wobei die Goldbeschichtung (30) eine Dicke von 0,5 bis 5 nm aufweist.

**3.** Glänzendes Pigment mit einem Goldton nach Anspruch 1,
wobei die Basisbeschichtung (20) eine silberhaltige Basisbeschichtung ist, die Silber enthält, und
die silberhaltige Basisbeschichtung (20) eine Dicke von 20 bis 100 nm aufweist.

**4.** Glänzendes Pigment mit einem Goldton nach Anspruch 1,
wobei die Basisbeschichtung (20) eine nickelhaltige Basisbeschichtung ist, die Nickel oder eine Legierung auf Nickelbasis enthält, und
die nickelhaltige Basisbeschichtung eine Dicke von 40 bis 200 nm aufweist.

**5.** Glänzendes Pigment nach Anspruch 1,
wobei die Legierung auf Nickelbasis eine Nickel-Phosphor-Legierung, eine Nickel-Wolfram-Phosphor-Legierung, eine Nickel-Eisen-Wolfram-Phosphor-Legierung, eine Nickel-Cobalt-Legierung, eine Nickel-Cobalt-Phosphor-Legierung, eine Nickel-Palladium-Phosphor-Legierung, eine Nickel-Zinn-Phosphor-Legierung oder eine Nickel-Kupfer-Phosphor-Legierung ist.

**6.** Glänzendes Pigment mit einem Goldton nach Anspruch 1,
wobei das Schuppenglas-Substrat (10) einen durchschnittlichen Partikeldurchmesser von 1 $\mu$m bis 500 $\mu$m und eine durchschnittliche Dicke von 0,3 $\mu$m bis 10 $\mu$m aufweist.

**7.** Kosmetikum, welches das glänzende Pigment mit einem Goldton nach einem der Ansprüche 1 bis 6 enthält.

**8.** Farbe, die das glänzende Pigment mit einem Goldton nach einem der Ansprüche 1 bis 6 enthält.

**9.** Druckfarbe, die das glänzende Pigment mit einem Goldton nach einem der Ansprüche 1 bis 6 enthält.

**10.** Harzzusammensetzung, die das glänzende Pigment mit einem Goldton nach einem der Ansprüche 1 bis 6 enthält.


**Revendications**

**1.** Pigment brillant à nuance dorée, comprenant :

un substrat de verre écailleux (10) ;
un revêtement de base (20) contenant au moins un matériau métallique choisi dans le groupe constitué de l'argent, du nickel et d'un alliage à base de nickel,
un revêtement d'or (30) contenant de l'or ; et
un revêtement jaune (40) contenant un colorant jaune et un matériau apparenté ;
dans lequel le colorant jaune est un colorant soluble dans l'eau ou un colorant insoluble dans l'eau, le colorant soluble dans l'eau est au moins un colorant choisi dans le groupe constitué des suivants : jaune acide 23, jaune aliment 3, jaune acide 73, jaune acide 3, jaune acide 40, jaune acide 1, jaune acide 36 et jaune acide 11, qui sont prescrits par le Color Index Generic Name, le colorant insoluble dans l'eau est au moins un colorant choisi dans le groupe constitué des suivants : jaune acide 73, jaune solvant 33, jaune pigment 12, jaune pigment 1, jaune solvant 5, jaune solvant 6, jaune pigment 110, jaune pigment 139, jaune pigment 150 et jaune pigment 154, qui sont prescrits par le Color Index Generic Name, lorsque le colorant jaune est un colorant soluble dans l'eau, le matériau apparenté comprend au moins un matériau choisi dans le groupe constitué de l'hydroxyde d'aluminium, de l'hydroxyde de zirconium, de l'hydroxyde de calcium, de l'oxyde d'aluminium hydraté, de l'oxyde de zirconium hydraté, de l'oxyde de calcium hydraté et de l'hydrotalcite, et le colorant soluble dans l'eau passe à travers le matériau apparenté dans le revêtement jaune, lorsque le colorant jaune est un colorant insoluble dans l'eau, le matériau parent comprend la silice, et le colorant insoluble dans l'eau est dispersé dans le matériau apparenté du revêtement jaune, et
le revêtement de base (20), le revêtement d'or (30) et le revêtement jaune (40) forment chacun une couche couvrant le substrat de verre écailleux (10), et le revêtement de base (20) est aménagé le plus à l'intérieur parmi le revêtement de base (20), le revêtement d'or (30) et le revêtement jaune (40).

**2.** Pigment brillant à nuance dorée selon la revendication 1,

dans lequel le revêtement d'or (30) a une épaisseur de 0,5 à 5 nm.

3. Pigment brillant à nuance dorée selon la revendication 1,
dans lequel le revêtement de base (20) est un revêtement de base contenant de l'argent, et
le revêtement de base contenant de l'argent (20) a une épaisseur de 20 à 100 nm.

4. Pigment brillant à nuance dorée selon la revendication 1,
dans lequel le revêtement de base (20) est un revêtement contenant du nickel ou un alliage à base de nickel, et
le revêtement de base contenant du nickel a une épaisseur de 40 à 200 nm.

5. Pigment brillant selon la revendication 1,
dans lequel l'alliage à base de nickel est un alliage de nickel-phosphore, un alliage de nickel-tungstène-phosphore, un alliage de nickel-fertungstène-phosphore, un alliage de nickel-cobalt, un alliage de nickel-cobalt-phosphore, un alliage de nickel-palladium-phosphore, un alliage de nickel-étain-phosphore ou un alliage de nickel-cuivre-phosphore.

6. Pigment brillant à nuance dorée selon la revendication 1,
dans lequel le substrat de verre écailleux (10) a un diamètre de particule moyen de 1 μm à 500 μm et une épaisseur moyenne de 0,3 μm à 10 μm.

7. Cosmétique contenant le pigment brillant à nuance dorée selon l'une quelconque des revendications 1 à 6.

8. Peinture contenant le pigment brillant à nuance dorée selon l'une quelconque des revendications 1 à 6.

9. Encre contenant le pigment brillant à nuance dorée selon l'une quelconque des revendications 1 à 6.

10. Composition de résine contenant le pigment brillant à nuance dorée selon l'une quelconque des revendications 1 à 6.

1

10    20    30    40    42

FIG. 1

2

10    20    40    42    30

FIG. 2

FIG. 3

FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002138010 A
- JP H9012919 A
- JP 2004352725 A
- WO 2005071019 A
- WO 0140383 A1
- US 20030072961 A1
- DE 2429762
- US 4084983 A

- JP 2002188023 A
- JP 2002194247 A
- JP 2001234090 A
- JP 2003213156 A
- JP H5171055 A
- JP H7133211 A
- JP 2006176742 A